# EUROPEAN PATENT APPLICATION

(11) **EP 4 520 299 A1**
(43) Date of publication of application: **12.03.2025**
(21) Application number: 22945382.4
(22) Date of filing: 17.06.2022
(51) Int. Cl.: A61F 2/24

(54) **VALVE DELIVERY SYSTEM**

(30) Priority: 09.06.2022 CN 202210649827; 09.06.2022 CN 202221437959 U
(71) Applicant: Kingstronbio (Changshu) Co., Ltd., Changshu Jiangsu 215500 (CN)
(72) Inventor: ZHONG, Shengping, Changshu, Jiangsu 215500 (CN); ZHANG, Bo, Changshu, Jiangsu 215500 (CN); MENG, Chunwang, Changshu, Jiangsu 215500 (CN)
(74) Representative: Luppi Intellectual Property S.r.l.
(86) International application number: PCT/CN2022/099408
(87) International publication number: WO 2023/236242

(57) **Abstract**

The present disclosure relates to a valve delivery system including a valve provided with fixing portions and a valve delivery device including a withdrawing assembly and a bolt wire assembly. The withdrawing assembly is provided with a plurality of fixing claws which have different sizes along an axial direction of the valve delivery device and are provided with limiting holes through which the fixing portions can pass. The bolt wire assembly is provided with a plurality of bolt wires having a same size along the axial direction of the valve delivery device. When the fixing portions pass through the limiting holes and the bolt wires pass through fixing holes of the fixing portion, the valve is in a connected state. When the bolt wires move along the axial direction of the valve delivery device, the bolt wires can be disengaged from the fixing claws having different lengths in sequence from long to short, respectively, until all the fixing claws are disengaged from the valve, and the valve is in a released state, which is used for realizing a stepwise release of the valve, thereby reducing the possibility of popping, displacing and injuring a human body when the valve is released, and improving the stability of release of the valve.

## Description

### TECHNICAL FIELD

The present disclosure relates to the technical field of medical instruments, and in particular, to a valve delivery system.

### BACKGROUND

With the development of medical technology, heart valve diseases can be treated by implanting heart valves. A self-expanding valve is an artificial valve that can curl up and shrink outside a body and can be automatically expanded and unfolded to achieve the effect of support and expansion after being delivered to a preset position in a human body through an implantation device. However, the valve disengages and bounces off the implantation device after being released and is prone to popping, causing the valve to displace and even injure the human body.

### SUMMARY

The present disclosure provides a valve delivery system for solving the problem of the displacement of a valve due to popping when it is released.

An embodiment of the present disclosure provides a valve delivery system, including a valve provided with a plurality of fixing portions and a valve delivery device, wherein the valve delivery device includes:
a withdrawing assembly provided with a plurality of fixing claws, where the plurality of fixing claws have different sizes along an axial direction of the valve delivery device and are provided with limiting holes, and the fixing portions are capable of passing through the limiting holes; and
a bolt wire assembly provided with a plurality of bolt wires, where the plurality of bolt wires have a same size along the axial direction of the valve delivery device;
where when the fixing portions pass through the limiting holes and the bolt wires pass through fixing holes of the fixing portions, the valve is in a connected state; and
when the bolt wires move along the axial direction of the valve delivery device, the bolt wires are capable of being disengaged from the fixing claws having different lengths from long to short, respectively, until all the fixing claws are disengaged from the fixing portions, the valve is in a released state.

The valve delivery system provided by the embodiment of the present disclosure is used to implant the valve, and the valve delivery system includes the valve and the valve delivery device, where the valve can be a self-expanding valve, and the valve delivery device can deliver the valve to a preset position in a human body. Upon the valve reaches the preset position, the valve delivery device can release the valve, and the valve can achieve the effect of expansion and support. Specifically, the valve is provided with the fixing portions, and the fixing portions are provided with the fixing holes. The valve delivery device includes the withdrawing assembly and the bolt wire assembly, where the withdrawing assembly is provided with the plurality of fixing claws, the bolt wire assembly is provided with the plurality of bolt wires, and the fixing claws are provided with the limiting holes. The plurality of fixing claws are provided along a circumferential direction of the withdrawing assembly for fixing the valve from different directions. At least a part of each of the fixing portions can pass through one of the limiting holes. At this point, the bolt wires pass through the fixing holes, respectively, thereby connecting the fixing portions and the fixing claws, and realizing the connection between the valve and the valve delivery device. At this point, the valve is in the connected state. When the valve delivery device delivers the valve to the preset position, the bolt wires can be disengaged from the fixing holes by moving the bolt wire assembly. Where the fixing claws have different sizes in the length direction of the valve delivery device, and the bolt wires have the same length in the length direction of the valve delivery device. Therefore, in the process of moving the bolt wires, the fixing portions connected to the fixing claws having different lengths are also released one after another. One bolt wire is first disengaged from the fixing claw with a longer length, and at the same time, the fixing portion connected to the fixing claw with the longer length is also first released. The different fixing portions are released in sequence with the movement of the bolt wire assembly, thereby realizing a stepwise release of the valve. Compared with a direct release manner commonly used for valve release in prior art, the valve and the valve delivery device may be directly disengaged from each other. Since the valve itself has a certain elasticity, the valve may be directly unfolded when it is released. This rapid deformation of larger magnitude may result in possible displacement of the valve relative to the preset position, and even injure the human body. However, the valve delivery device provided by the embodiment of the present disclosure can achieve the stepwise release of the valve, thereby reducing the magnitude of deformation when the valve is released, thereby facilitating the control over the implantation of the valve and reducing the possibility of the displacement of the valve and the injury to the human body.

In a possible implementation, the withdrawing assembly is provided with four fixing claws, which are provided along a circumferential direction of the withdrawing assembly and include a first fixing claw, a second fixing claw, a third fixing claw, and a fourth fixing claw; and a length of the first fixing claw is greater than a length of the second fixing claw, the length of the second fixing claw is greater than a length of the third fixing claw, and a length of the fourth fixing claw is equal to the length of the second fixing claw.

The plurality of fixing claws are provided along the circumferential direction of the withdrawing assembly, and the fixing claws includes the first fixing claw, the second fixing claw, the third fixing claw, and the fourth fixing claw. The fixing claws can be connected to the fixing portions of the valve from different directions at the same time, thereby stabilizing the valve when it is in the connected state. Specifically, the lengths of the fixing claws are different, when the bolt wire assembly moves to disengage the bolt wires from the fixing holes: the valve is separated from the first fixing claw, causing the valve to be partially released; the second fixing claw and the fourth fixing claw have the same length, the valve is disengaged from both the second fixing claw and the fourth fixing claw, causing both sides of the valve to be simultaneously unfixed, reducing the possibility of the valve deviating to one side when it is released; and finally, the valve is disengaged from the third fixing claw having the shortest length, and the valve is completely released. The embodiment of the present application realizes stepwise disengagement of the valve from the fixing claws by providing the fixing claws of different lengths, which reduces the magnitude of deformation of the valve at each step of release, makes the release of the valve more stable, and reduces the possibility of popping when the valve is released.

In a possible implementation, the first fixing claw, the second fixing claw, the third fixing claw, and the fourth fixing claw are provided in sequence along the circumferential direction of the withdrawing assembly.

When the valve is released, the first fixing claw is first disengaged from the valve, causing a part of the valve to be released. At this point, the second fixing claw, the third fixing claw, the fourth fixing claw are still connected to the valve. The third fixing claw is provided adjacent to the second fixing claw and the fourth fixing claw, respectively, and the third fixing claw is provided between the second fixing claw and the fourth fixing claw. The second fixing claw and the fourth fixing claw have the same length and fix the valve from both sides of the valve, respectively, so that the valve can remain stable after the first fixing claw is released. Furthermore, when being released, the second fixing claw and the fourth fixing claw release the restriction of the valve from both sides of the valve, respectively, making the force on the valve when it is released more balanced and the release more stable.

In a possible implementation, the valve delivery device includes a wire release device and a wire release connecting tube, one end of the wire release connecting tube is connected to the bolt wire assembly, the other end of the wire release connecting tube is connected to the wire release device, and the wire release device is movable along the axial direction of the valve delivery device.

The wire release device is provided with a handle and is connected to the bolt wire assembly through the wire release connecting tube. When the valve delivery device delivers the valve into the human body, the control over the movement of the bolt wires is facilitated by the wire release device, thereby achieving that the movement of the bolt wires control the release of the valve.

In a possible implementation, the valve delivery device includes a locking device capable of locking the wire release device for limiting the movement of the wire release device along the axial direction of the valve delivery device.

The locking device is provided at an end of the wire release device away from the bolt wire assembly. The locking device may include a locking groove and a locking member. A part of the locking member may extend into the locking groove, and another part of the locking member protrudes relative to the locking groove, thereby limiting the wire release device from moving in a direction away from the bolt wire assembly. Upon the locking member is disengaged from the locking groove, the locking of the wire release device is released. The locking device can reduce the possibility of misoperation and reduce the risk that the valve is prematurely released.

In a possible implementation, the locking device includes a first locking member and a second locking member that are provided along the axial direction of the valve delivery device, and a distance between the first locking member and the wire release device is less than a distance between the second locking member and the wire release device.

The locking device is provided with the first locking member and the second locking member, and the distance between the first locking member and the wire release device is less than the distance between the second locking member and the wire release device, which may be achieved specifically by setting the sizes of the first locking member and the second locking member, respectively, or setting the first locking member and the second locking member at different mounting positions in the locking device, respectively. The first locking member and the second locking member limit the movement of the wire release device by abutting against the wire release device to achieve the effect of locking. When the first locking member is released, the wire release device can move to a position where it abuts against the second locking member to partially release the valve. When the second locking member is released, the wire release device can continue to move to completely release the valve. By providing the first locking member and the second locking member, the stepwise release of the valve can be released, reducing the possibility of popping when the valve is released, and at the same time, reducing the situation of premature release of the valve due to misoperation when the valve is released. For example, when the wire release device abuts against the first locking member, the valve cannot be released and is in the connected state. When the first locking member is released, the wire release device can move to separate the valve from the first fixing claw, causing the valve to be partially released. When the second locking member is released, the wire release device can continue to move, so that the valve is simultaneously disengaged from the second fixing claw and the fourth fixing claw, and finally disengaged from the third fixing claw, causing the valve to be completely released.

In a possible implementation, the withdrawing assembly is provided with through holes, and the bolt wires are capable of passing through the through holes and the fixing holes.

The bolt wire assembly can move along the axial direction of the valve delivery device. Where the bolt wire assembly is provided on a side of the withdrawing assembly away from the valve. The through holes pass through the withdrawing assembly along the axial direction of the valve delivery device. The bolt wires can pass through the through holes and extend into the fixing holes of the valve, thereby limiting the valve from being unhooked, and causing the valve in the connected state. When the bolt wire assembly moves towards a direction away from the withdrawing assembly, the bolt wires can move in the through holes to be disengaged from the fixing holes, the valve is no longer limited by the bolt wires, and the valve can be unfolded under its own elastic force. The through holes are provided corresponding to the fixing claws. The through holes can play a guiding role for the bolt wires, so that the bolt wires can be accurately matched with the fixing holes of the valve, thereby reducing the possibility that the bolt wires deviate.

In a possible implementation, the withdrawing assembly is provided with mounting grooves, the fixing claws are provided in the mounting grooves, and the mounting grooves are in communication with the avoidance portions and are provided corresponding to the through holes.

The mounting grooves are provided corresponding to the fixing claws, and the mounting grooves are in communication with the avoidance portions. When the valve is in the connected state, at least a part of each of the fixing claws can be embedded into one of the mounting grooves, so that the fixing claws do not protrude relative to the surface of the withdrawing assembly, which facilitates the storage of the withdrawing assembly, thereby facilitating the valve delivery device to deliver the valve into the human body; and further, the limiting holes can be located in the avoidance portions, facilitating the fixing portions to extend into the limiting holes to match with the bolt wires.

In a possible implementation, the withdrawing assembly is provided with avoidance portions that are provided at an end of the withdrawing assembly away from the bolt wire assembly, the avoidance portions are configured to provide spaces for the fixing portions to extend into the limiting holes, and are in communication with the through holes.

The avoidance portions are provided in the withdrawing assembly and on a side of the fixing claws close to the axial direction of the withdrawing assembly, and the avoidance portions enable the fixing claws to extend from the mounting grooves. When the fixing portions extend into the limiting holes, the fixing portions can be bent along a direction close to the axis of the withdrawing assembly, and at least a part of each of the fixing portions can extend into one of the avoidance portions, so that the fixing holes can be matched with the bolt wires.

In a possible implementation, the valve delivery system includes an outer tube and a rotating wheel assembly movable along the axial direction of the valve delivery device for driving the outer tube to move to release the valve.

The outer tube is configured to fixedly store the valve. The rotating wheel assembly is provided at an end of the delivery system away from the valve. The rotating wheel assembly includes threads. When the rotating wheel assembly is rotated, the rotating wheel assembly can move along the axial direction of the valve delivery device, thereby driving the outer tube to move to release the restriction on the valve and thus to release the valve. The control using the rotating wheel assembly can improve the precision of releasing the outer tube.

The embodiment of the present disclosure provides a valve delivery system. The valve delivery system includes the valve provided with the fixing portions and the valve delivery device. The valve delivery device includes the withdrawing assembly and the bolt wire assembly. The withdrawing assembly is provided with the plurality of fixing claws. The fixing claws have different sizes along the axial direction of the valve delivery device, the fixing claws are provided with the limiting holes, and the fixing portions can pass through the limiting holes. The bolt wire assembly is provided with the plurality of bolt wires. The bolt wires have the same size along the axial direction of the valve delivery device. Where when the fixing portions pass through the limiting holes and the bolt wires pass through the fixing holes of the fixing portions, the valve is in the connected state. When the bolt wires move along the axial direction of the valve delivery device, the bolt wires can be disengaged from the fixing claws having different lengths from long to short, respectively, until all the fixing claws are disengaged from the valve, and the valve is in the released state, which is used for realizing the stepwise release of the valve, thereby reducing the possibility of popping, displacing and injuring the human body when the valve is released, and improving the stability of release of the valve.

It should be understood that the general description above and the detailed description in the following are merely exemplary, and cannot limit the present disclosure.

### BRIEF DESCRIPTION OF DRAWINGS

FIG. 1 is a structural schematic diagram of a valve delivery device provided by the present disclosure;
FIG. 2 is a structural schematic diagram of a bolt wire assembly provided by the present disclosure;
FIG. 3 is a structural schematic diagram of a withdrawing assembly provided by the present disclosure;
FIG. 4 is a structural schematic diagram of connections between fixing portions and fixing claws provided by the present disclosure;
FIG. 5 is a structural schematic diagram of a wire release device provided by the present disclosure; and
FIG. 6 is a structural schematic diagram of an introduction device provided by the present disclosure.

### Reference signs:

1-fixing portion;
11-fixing hole;
2-bolt wire assembly;
   21-bolt wire;
3-withdrawing assembly;
   31-fixing claw;
      311-first fixing claw;
      312-second fixing claw;
      313-third fixing claw;
      314-fourth fixing claw;
   32-limiting hole;
   33-through hole;
   34-avoidance portion;
   35-mounting groove;
   4-wire release device;
   5-wire release connecting tube;
   6-outer tube;
   7-rotating wheel assembly;
   8-locking device;
   81-first locking member;
   82-second locking member;
   9-introduction device;
   91-protrusion.

The accompanying drawings herein, which are incorporated in and constitute a part of this specification of the present disclosure, show embodiments consistent with the present disclosure and, together with the specification, serve to explain principles of the present disclosure.

### DESCRIPTION OF EMBODIMENTS

To better understand the technical solutions of the present disclosure, embodiments of the present disclosure are described in detail below in conjunction with the accompanying drawings.

It should be clear that the described embodiments are only some embodiments of the present disclosure, not all embodiments of the present disclosure. Based on the embodiments in the present disclosure, all other embodiments obtained by those of ordinary skill in the art without creative efforts shall fall within the protection scope of the present disclosure.

The terms used in the embodiments of the present disclosure are merely for the purpose of describing particular embodiments but not intended to limit the present disclosure. Unless otherwise noted in the context, the singular form expressions "a/an", "said" and "the" used in the embodiments and appended claims of the present disclosure are also intended to include plural form expressions thereof.

It should be understood that the term "and/or" used herein is merely an association relationship describing associated objects, indicating that there may be three relationships, for example, A and/or B may indicate three cases, i.e., A existing individually, A and B existing simultaneously, B existing individually. In addition, the character "/" herein generally indicates that the associated objects before and after the character form an "or" relationship.

It should be noted that, the words for orientation such as "upper", "lower", "left", "right" and the like as mentioned in the embodiments of the present disclosure are described with reference to the placement status in the accompanying drawings, and should not be construed as limiting the embodiments of the present disclosure. In addition, it should also be understood that, in the context, while referring to an element being formed "above" or "below" another element, it is possible that the element is directly formed "above" or "below" the another element, it is also possible that the element is formed "above" or "below" the another element via an intermediate element.

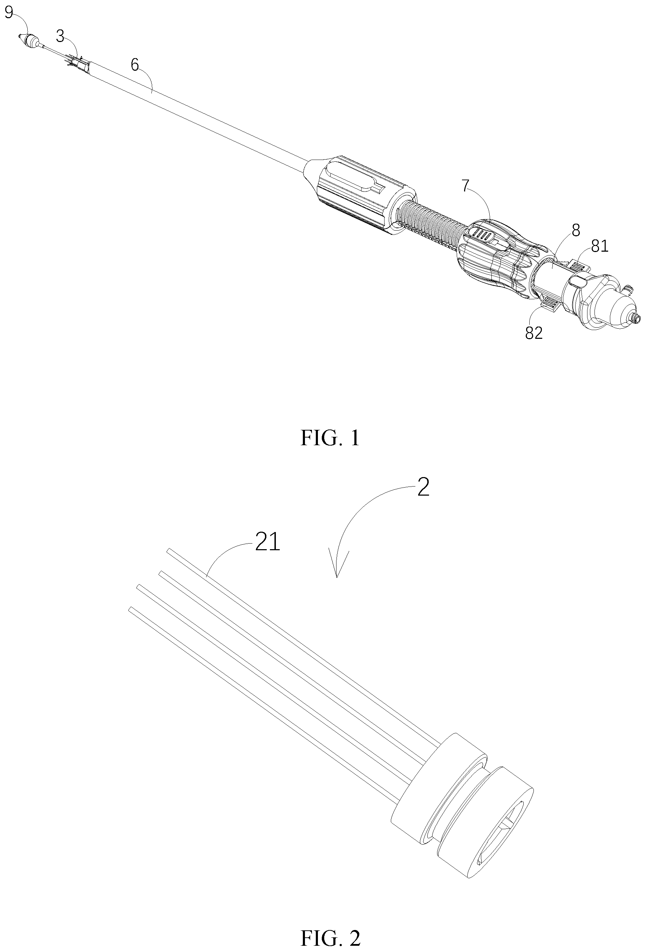

As shown in FIG. 1 to FIG. 4, an embodiment of the present disclosure provides a valve delivery system. The valve delivery system includes a valve provided with fixing portions 1 and a valve delivery device. The valve delivery device includes a withdrawing assembly 3 and a bolt wire assembly 2. The withdrawing assembly 3 is provided with a plurality of fixing claws 31. The fixing claws 31 have different sizes along an axial direction of the valve delivery device and are provided with limiting holes 32. The fixing portions 1 can pass through the limiting holes 32. The bolt wire assembly 2 is provided with a plurality of bolt wires 21, and the bolt wires 21 have a same size along the axial direction of the valve delivery device. Where when the fixing portions 1 pass through the limiting holes 32 and the bolt wires 21 pass through fixing holes 11 of the fixing portions 1, the valve is in a connected state. When the bolt wires 21 move along the axial direction of the valve delivery device, the bolt wires 21 can be disengaged from the fixing claws 31 having different lengths in sequence from long to short, respectively, until all the fixing claws 31 are disengaged from the fixing portions, the valve is in a released state.

The valve delivery system provided by the embodiment of the present disclosure is used to implant the valve, and the valve delivery system includes the valve and the valve delivery device. Where the valve can be a self-expanding valve, and the valve delivery device can deliver the valve to a preset position in a human body. Upon the valve reaches the preset position, the valve delivery device can release the valve, and the valve can achieve the effect of expansion and support. Specifically, the valve has the fixing portions 1, and the fixing portions 1 are provided with the fixing holes 11. The valve delivery device includes the withdrawing assembly 3 and the bolt wire assembly 2. Where the withdrawing assembly 3 is provided with the plurality of fixing claws 31, the bolt wire assembly 2 is provided with the plurality of bolt wires 21, and the fixing claws 31 are provided with the limiting holes 32. The plurality of fixing claws 31 are provided along a circumferential direction of the withdrawing assembly 3 for fixing the valve from different directions. At least a part of the fixing portion 1 can pass through the limiting hole 32. At the point, the bolt wires 21 pass through the fixing holes 11, respectively, thereby connecting the fixing portions 1 with the fixing claws 31, and realizing the connection between the valve and the valve delivery device. At this point, the valve is in the connected state. When the valve delivery device delivers the valve to the preset position, the bolt wires 21 can be disengaged from the fixing holes 11 by moving the bolt wire assembly 2. Where the fixing claws 31 have different sizes in the length direction of the valve delivery device, while the bolt wires have the same length. Therefore, in the process of moving the bolt wires 21, the fixing portions 1 connected with the fixing claws 31 having different lengths are released in sequence. The bolt wire 21 is firstly disengaged from the fixing claw 31 with a longer length, and at the same time, the fixing portion 1 connected with the fixing claw 31 with a longer length is also released firstly. A different fixing portion 1 is released in sequence with the movement of the bolt wire assembly 2, thereby realizing a stepwise release of the valve. Compared with a direct release manner used for valve release in prior art, the valve and the valve delivery device may be directly disengaged from each other. Since the valve itself has a certain elasticity, the valve may be directly unfolded when it is released. This rapid deformation of larger magnitude may result in possible displacement of the valve relative to the preset position, and even injure the human body. However, the valve delivery device provided by the embodiment of the present disclosure can achieve the stepwise release of the valve, thereby reducing the magnitude of deformation when the valve is released, thereby facilitating the control over the implantation of the valve and reducing the possibility of the displacement of the valve and the injury to the human body.

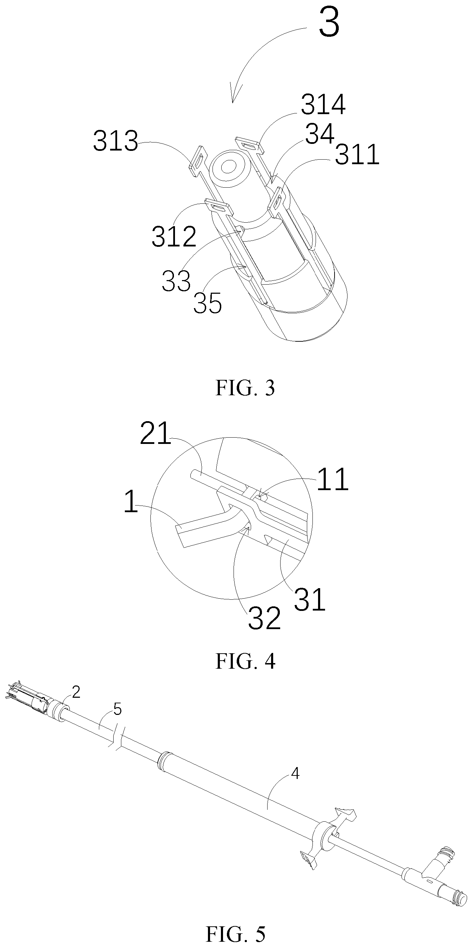

As shown in FIG. 3, in a possible implementation, the withdrawing assembly 3 is provided with four fixing claws 31, and the fixing claws 31 are provided along the circumferential direction of the withdrawing assembly 3. The fixing claws 31 include a first fixing claw 311, a second fixing claw 312, a third fixing claw 313, and a fourth fixing claw 314. A length of the first fixing claw 311 is greater than a length of the second fixing claw 312, the length of the second fixing claw 312 is greater than a length of the third fixing claw 313, and a length of the fourth fixing claw 314 is equal to the length of the second fixing claw 312.

The plurality of fixing claws 31 are provided along the circumferential direction of the withdrawing assembly 3. The fixing claws 31 includes the first fixing claw 311, the second fixing claw 312, the third fixing claw 313, and the fourth fixing claw 314. The fixing claws 31 can be connected to the fixing portions 1 of the valve from different directions at the same time, thereby stabilizing the valve when it is in the connected state. Specifically, the lengths of the fixing claws 31 are different, when the bolt wire assembly 2 moves to disengage the bolt wires 21 from the fixing holes 11, the valve is separated from the first fixing claw 311, causing the valve to be partially released. The second fixing claw 312 and the fourth fixing claw 314 have the same length, and the valve is disengaged from both the second fixing claw 312 and the fourth fixing claw 314, causing both sides of the valve to be simultaneously unfixed, reducing the possibility of the valve deviating to one side when it is released. Finally, the valve is disengaged from the third fixing claw 313 having the shortest length, and the valve is completely released. The embodiment of the present application realizes stepwise disengagement of the valve from the fixing claws 31 by providing the fixing claws 31 of different lengths, which reduces the magnitude of deformation of the valve at each step of release, makes the release of the valve more stable, and reduces the possibility of popping when the valve is released.

As shown in FIG. 3, in a possible implementation, the first fixing claw 311, the second fixing claw 312, the third fixing claw 313, and the fourth fixing claw 314 are sequentially provided along the circumferential direction of the withdrawing assembly 3.

When the valve is released, the first fixing claw 311 is first disengaged from the valve, causing a part of the valve to be released. At this point, the second fixing claw 312, the third fixing claw 313, the fourth fixing claw 314 are still connected to the valve. The third fixing claw 313 is provided adjacent to the second fixing claw 312 and the fourth fixing claw 314, respectively, and the third fixing claw 313 is provided between the second fixing claw 312 and the fourth fixing claw 314. The second fixing claw 312 and the fourth fixing claw 314 have the same length and fix the valve from both sides of the valve, respectively, so that the valve can remain stable after the first fixing claw 311 is released. Furthermore, when being released, the second fixing claw 312 and the fourth fixing claw 314 release the restriction of the valve from both sides of the valve, respectively, making the force on the valve when it is released more balanced and the release more stable.

As shown in FIG. 5, in a possible implementation, the valve delivery device includes a wire release device 4 and a wire release connecting tube 5. One end of the wire release connecting tube 5 is connected to the bolt wire assembly 2, and another end of the wire release connecting tube 5 is connected to the wire release device 4. The wire release device 4 is movable along the axial direction of the valve delivery device.

The wire release device 4 is provided with a handle and is connected to the bolt wire assembly 2 through the wire release connecting tube 5. When the valve delivery device delivers the valve into the human body, the control over the movement of the bolt wires 21 is facilitated by the wire release device 4, thereby achieving that the movement of the bolt wires 21 control the release of the valve.

As shown in FIG. 1, in a possible implementation, the valve delivery device includes a locking device 8 capable of locking the wire release device 4 and limiting the movement of the wire release device 4 along the axial direction of the valve delivery device.

The locking device 8 is provided at an end of the wire release device 4 away from the bolt wire assembly 2. The locking device 8 may include a locking groove and a locking member. A part of the locking member may extend into the locking groove, and another part of the locking member protrudes relative to the locking groove, thereby limiting the wire release device 4 from moving in a direction away from the bolt wire assembly 2. Upon the locking member is disengaged from the locking groove, the locking of the wire release device 4 is released. The locking device 8 can reduce the possibility of misoperation and reduce the risk that the valve is prematurely released.

The wire release device 4 may also be provided as a rotating wheel that can move along the axial direction of the valve delivery device. The movement of the wire release device 4 along the axial direction of the valve delivery device is realized by rotating the rotating wheel, thereby achieving the effects of reducing misoperation and locking.

As shown in FIG. 1, in a possible implementation, the locking device 8 includes a first locking member 81 and a second locking member 82 that are provided along the axial direction of the valve delivery device, and a distance between the first locking member 81 and the wire release device 4 is less than a distance between the second locking member 82 and the wire release device 4.

The locking device 8 is provided with the first locking member 81 and the second locking member 82, and the distance between the first locking member 81 and the wire release device 4 is less than the distance between the second locking member 82 and the wire release device 4, which may be achieved specifically by setting the sizes of the first locking member 81 and the second locking member 82, respectively, or setting the first locking member 81 and the second locking member 82 at different mounting positions in the locking device 8, respectively. The first locking member 81 and the second locking member 82 limit the movement of the wire release device 4 by abutting against the wire release device 4 to achieve the effect of locking. When the first locking member 81 is released, the wire release device 4 can move to a position where it abuts against the second locking member 82 to partially release the valve. When the second locking member 82 is released, the wire release device 4 can continue to move to completely release the valve. By providing the first locking member 81 and the second locking member 82, the stepwise release of the valve can be released, reducing the possibility of popping when the valve is released, and at the same time, reducing the situation of premature release of the valve due to misoperation when the valve is released. For example, when the wire release device 4 abuts against the first locking member 81, the valve cannot be released and is in the connected state. When the first locking member 81 is released, the wire release device 4 can move to separate the valve from the first fixing claw 311, causing the valve to be partially released. When the second locking member 82 is released, the wire release device 4 can continue to move, so that the valve is simultaneously disengaged from the second fixing claw 312 and the fourth fixing claw 314, and finally disengaged from the third fixing claw 313, causing the valve to be completely released.

As shown in FIG. 3, in a possible implementation, the withdrawing assembly 3 is provided with through holes 33, and the bolt wires 21 can pass through the through holes 33 and the fixing holes 11.

The bolt wire assembly 2 can move along the axial direction of the valve delivery device. Where the bolt wire assembly 2 is provided on a side of the withdrawing assembly 3 away from the valve. The through holes 33 pass through the withdrawing assembly 3 along the axial direction of the valve delivery device. The bolt wires 21 can pass through the through holes 33 and extend into the fixing holes 11 of the valve, thereby limiting the valve from being unhooked, and causing the valve in the connected state. When the bolt wire assembly 2 moves towards a direction away from the withdrawing assembly 3, the bolt wires 21 can move in the through holes 33 to be disengaged from the fixing holes 11, the valve is no longer limited by the bolt wires 21, and the valve can be unfolded under its own elastic force. The through holes 33 are provided corresponding to the fixing claws 31. The through holes 33 can play a guiding role for the bolt wires 21, so that the bolt wires 21 can be accurately matched with the fixing holes 11 of the valve, thereby reducing the possibility that the bolt wires 21 deviate.

As shown in FIG. 3, in a possible implementation, the withdrawing assembly 3 is provided with mounting grooves 35. The fixing claws 31 are provided in the mounting grooves 35, and the mounting grooves 35 are provided corresponding to the through holes 33.

The mounting grooves 35 are provided corresponding to the fixing claws 31. When the valve is in the connected state, at least a part of each of the fixing claws 31 can be embedded into one of the mounting grooves 35, so that the fixing claws 31 do not protrude relative to the surface of the withdrawing assembly 3, which facilitates the storage of the withdrawing assembly 3, thereby facilitating the valve delivery device to deliver the valve into the human body; and further, the limiting holes 32 can be located in the avoidance portions 34, facilitating the fixing portions 1 to extend into the limiting holes 32 to match with the bolt wires 21.

As shown in FIG. 3, in a possible implementation, the withdrawing assembly 3 is provided with the avoidance portions 34. The avoidance portions 34 are provided at an end of the withdrawing assembly 3 away from the bolt wire assembly 2, are configured to provide spaces for the fixing portions 1 to extend into the limiting holes 32, and are in communication with the through holes 33.

The avoidance portions 34 are provided in the withdrawing assembly 3 and on a side of the fixing claws 31 close to the axial direction of the withdrawing assembly 3, and the avoidance portions 34 enable the fixing claws 31 to extend from the mounting grooves 35. When the fixing portions 1 extend into the limiting holes 32, the fixing portions 1 can be bent along a direction close to the axis of the withdrawing assembly 3, and at least a part of each of the fixing portions 1 can extend into one of the avoidance portions 34, so that the fixing holes 11 can be matched with the bolt wires 21.

As shown in FIG. 1, in a possible implementation, the valve delivery system includes an outer tube 6 and a rotating wheel assembly 7. The rotating wheel assembly 7 is movable along the axial direction of the valve delivery device for driving the outer tube 6 to move to release the valve.

The outer tube 6 is configured to fixedly store the valve. The rotating wheel assembly 7 is provided at an end of the delivery system away from the valve. The rotating wheel assembly 7 includes threads. When the rotating wheel assembly 7 is rotated, the rotating wheel assembly 7 can move along the axial direction of the valve delivery device, thereby driving the outer tube 6 to move to release the restriction on the valve and thus to release the valve. The control using the rotating wheel assembly 7 can improve the precision of releasing the outer tube 6.

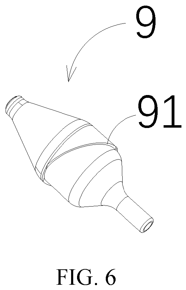

As shown in FIG. 6, in a possible embodiment, the valve delivery device includes an introduction device 9. The introduction device 9 is provided at an end of the valve delivery device away from the locking device 8 for introducing the valve delivery device into the human body when the valve is implanted.

The introduction device 9 has a conical tip to facilitate entry into the human body. Where the introduction device 9 includes a protrusion 91 provided around the tip and having an arc shape, which is consistent with the shape of an end portion of the valve in the connected state to facilitate the storage of the valve by the valve delivery device. An opening of the outer tube 6 can further be provided as a corresponding arc opening, which is matched with the protrusion 91 of the introduction device 9, to facilitate the introduction of the valve delivery device into the human body by the introduction device 9.

The embodiment of the present disclosure provides a valve delivery system. The valve delivery system includes the valve provided with the fixing portions 1 and the valve delivery device. The valve delivery device includes the withdrawing assembly 3 and the bolt wire assembly 2. The withdrawing assembly 3 is provided with the plurality of fixing claws 31. The fixing claws 31 have different sizes along the axial direction of the valve delivery device, the fixing claws 31 are provided with the limiting holes 32, and the fixing portions 1 can pass through the limiting holes 32. The bolt wire assembly 2 is provided with the plurality of bolt wires 21. The bolt wires 21 have the same size along the axial direction of the valve delivery device. Where when the fixing portions 1 pass through the limiting holes 32 and the bolt wires 21 pass through the fixing holes 11 of the fixing portions 1, the valve is in the connected state. When the bolt wires 21 move along the axial direction of the valve delivery device, the bolt wires 21 can be disengaged from the fixing claws having different lengths from long to short, respectively, until all the fixing claws 31 are disengaged from the valve, and the valve is in the released state, which is used for realizing the stepwise release of the valve, thereby reducing the possibility of popping, displacing and injuring the human body when the valve is released, and improving the stability of release of the valve.

The above description are merely preferred embodiments of the present disclosure and are not intended to limit the present disclosure. Various changes and modifications can be made to the present disclosure by those skilled in the art. Any modifications, equivalent substitutions and improvements made within the spirit and principle of the present disclosure shall fall within the protection scope of the present disclosure.

## Claims

1. A valve delivery system, comprising a valve provided with a plurality of fixing portions (1) and a valve delivery device, wherein the valve delivery device comprises:
a withdrawing assembly (3) provided with a plurality of fixing claws (31), wherein the plurality of fixing claws (31) have different sizes along an axial direction of the valve delivery device and are provided with limiting holes (32), and the fixing portions (1) are capable of passing through the limiting holes (32); and
a bolt wire assembly (2) provided with a plurality of bolt wires (21), wherein the plurality of bolt wires (21) have a same size along the axial direction of the valve delivery device;
wherein when the fixing portions (1) pass through the limiting holes (32) and the bolt wires (21) pass through fixing holes (11) of the fixing portions (1), the valve is in a connected state; and
when the bolt wires (21) move along the axial direction of the valve delivery device, the bolt wires (21) are capable of being disengaged from the fixing claws (31) having different lengths from long to short, respectively, until all the fixing claws (31) are disengaged from the fixing portions (1), the valve is in a released state.

2. The valve delivery system according to claim 1, wherein the withdrawing assembly (3) is provided with four fixing claws (31), which are provided along a circumferential direction of the withdrawing assembly (3) and comprise a first fixing claw (311), a second fixing claw (312), a third fixing claw (313), and a fourth fixing claw (314); and a length of the first fixing claw (311) is greater than a length of the second fixing claw (312), the length of the second fixing claw (312) is greater than a length of the third fixing claw (313), and a length of the fourth fixing claw (314) is equal to the length of the second fixing claw (312).

3. The valve delivery system according to claim 2, wherein the first fixing claw (311), the second fixing claw (312), the third fixing claw (313), and the fourth fixing claw (314) are provided in sequence along the circumferential direction of the withdrawing assembly (3).

4. The valve delivery system according to claim 1, wherein the valve delivery device comprises a wire release device (4) and a wire release connecting tube (5), one end of the wire release connecting tube (5) is connected to the bolt wire assembly (2), the other end of the wire release connecting tube (5) is connected to the wire release device (4), and the wire release device (4) is movable along the axial direction of the valve delivery device.

5. The valve delivery system according to claim 4, wherein the valve delivery device comprises a locking device (8) capable of locking the wire release device (4) for limiting the movement of the wire release device (4) along the axial direction of the valve delivery device.

6. The valve delivery system according to claim 5, wherein the locking device (8) comprises a first locking member (81) and a second locking member (82) that are provided along the axial direction of the valve delivery device, and a distance between the first locking member (81) and the wire release device (4) is less than a distance between the second locking member (82) and the wire release device (4).

7. The valve delivery system according to claim 1, wherein the withdrawing assembly (3) is provided with through holes (33), and the bolt wires (21) are capable of passing through the through holes (33) and the fixing holes (11).

8. The valve delivery system according to claim 7, wherein the withdrawing assembly (3) is provided with avoidance portions (34) that are provided at an end of the withdrawing assembly (3) away from the bolt wire assembly (2), the avoidance portions (34) are configured to provide spaces for the fixing portions (1) to extend into the limiting holes (32), and are in communication with the through holes (33).

9. The valve delivery system according to claim 8, wherein the withdrawing assembly (3) is provided with mounting grooves (35), the fixing claws (31) are provided in the mounting grooves (35), and the mounting grooves (35) are in communication with the avoidance portions (34) and are provided corresponding to the through holes (33).

10. The valve delivery system according to any one of claims 1 to 9, wherein the valve delivery system comprises an outer tube (6) and a rotating wheel assembly (7) movable along the axial direction of the valve delivery device for driving the outer tube (6) to move to release the valve.
